(19) Europäisches Patentamt
European Patent Office
Office européen des brevets

(11) Veröffentlichungsnummer: **0 453 883 A2**

(12) **EUROPÄISCHE PATENTANMELDUNG**

(21) Anmeldenummer: **91105745.3**

(22) Anmeldetag: **11.04.91**

(51) Int. Cl.5: **C07D 213/74**, C07D 213/75, C07D 213/76, C07F 9/58, C07D 401/04

(30) Priorität: **23.04.90 DE 4012831**

(43) Veröffentlichungstag der Anmeldung: **30.10.91 Patentblatt 91/44**

(84) Benannte Vertragsstaaten: **BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Hagen, Helmut, Dr.**
**Max-Slevogt-Strasse 17 e**
**W-6710 Frankenthal(DE)**
Erfinder: **Ziegler, Hans, Dr.**
**Pfalzring 91**
**W-6704 Mutterstadt(DE)**
Erfinder: **Pfister, Juergen, Dr.**
**Ziegelofenweg 1**
**W-6720 Speyer(DE)**
Erfinder: **Nilz, Gerhard, Dr.**
**Haardtstrasse 13**
**W-6701 Dannstadt-Schauernheim(DE)**
Erfinder: **Lorenz, Gisela, Dr.**
**Erlenweg 13**
**W-6730 Neustadt(DE)**
Erfinder: **Dressel, Juergen, Dr.**
**Medenheimer Strasse 18**
**W-6708 Neuhofen(DE)**

(54) **2-Amino-4-trichlorpyridinderivate.**

(57) 2-Amino-4-trichlormethylpyridine der allgemeinen Formel I

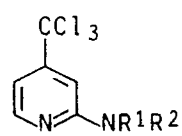

I

in der die Substituenten folgende Bedeutung haben:

| | |
|---|---|
| $R^1$ | Wasserstoff oder $C_1$-$C_8$-Alkyl; |
| $R^2$ | $C_1$-$C_8$-Alkyl; $C_5$-$C_8$-Cycloalkyl; $C_2$-$C_4$-Alkenyl; gegebenenfalls substituiertes Phenyl, Benzyl oder Phenylsulfonyl; -CX-$R^3$, -$SO_2R^3$ oder -$PX(OR^4)_2$ oder |
| $R^1$ und $R^2$ | gemeinsam $=CR^5R^6$ oder -CO-Z-CO-, mit |
| X | Sauerstoff oder Schwefel, |
| $R^3$ | $C_1$-$C_{20}$-Alkyl; $C_2$-$C_6$-Alkenyl; Amino; $C_1$-$C_6$-Alkylamino; Di-$C_1$-$C_6$-alkylamino; Morpholino; Piperidino; Pyrazolidino; $C_1$-$C_8$-Alkylcarbonylamino; gegebenenfalls substituiertes Phenylamino oder Benzylamino; gegebenenfalls substituiertes Phenyl oder Heteroaryl; |
| $R^4$ | $C_1$-$C_8$-Alkyl; $C_1$-$C_8$-Halogenalkyl oder gegebenenfalls substituiertes Phenyl; |
| $R^5$ | Wasserstoff; $C_1$-$C_4$-Alkyl oder gegebenenfalls substituiertes Phenyl; |
| $R^6$ | gegebenenfalls substituiertes Phenyl; |
| Z | gegebenenfalls substituiertes Ethylen, welches Teil eines gegebenenfalls substituierten |

EP 0 453 883 A2

Cycloalkyl- oder Cycloalkylenrestes sein kann;
gegebenenfalls substituiertes Ethenylen, welches Teil eines gegebenenfalls substituierten aromatischen oder heteroaromatischen Ringes sein kann;
wobei Z nicht Teil eines unsubstituierten Phenylrestes ist, sowie deren landwirtschaftlich brauchbaren Salze, Verfahren zu ihrer Herstellung und ihre Verwendung als Nitrifikationsinhibitoren.

Die vorliegende Erfindung betrifft 2-Amino-4-trichlormethylpyridine der allgemeinen Formel I

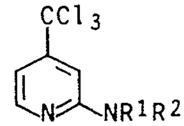

I

in der die Substituenten folgende Bedeutung haben:

$R^1$ Wasserstoff oder eine $C_1$-$C_8$-Alkylgruppe;

$R^2$ eine $C_1$-$C_8$-Alkylgruppe; eine $C_5$-$C_8$-Cycloalkylgruppe; eine $C_2$-$C_4$-Alkenylgruppe; einen Phenyl-, Benzyl- oder Phenylsulfonylrest, wobei die aromatischen Reste ihrerseits ein- bis fünffach durch Halogen und ein- bis dreifach durch folgende Gruppen substituiert sein können: $C_1$-$C_4$-Alkyl, $C_1$-$C_4$-Halogenalkyl, $C_1$-$C_4$-Alkoxy, $C_2$-$C_4$-Halogenalkoxy und $C_1$-$C_4$-Alkylthio, oder wobei die aromatischen Reste ihrerseits ein- bis fünffach durch Halogen oder ein- bis dreifach durch folgende Gruppen substituiert sein können: $C_1$-$C_4$-Alkyl, $C_1$-$C_4$-Halogenalkyl, $C_1$-$C_4$-Alkoxy, $C_2$-$C_4$-Halogenalkoxy und $C_1$-$C_4$-Alkylthio;
einen Rest $-CX-R^3$, $-SO_2R^3$ oder $-PX(OR^4)_2$ oder

$R^1$ und $R^2$ gemeinsam $=CR^5R^6$ oder -CO-Z-CO-, wobei

X für Sauerstoff oder Schwefel steht,

$R^3$ $C_1$-$C_{20}$-Alkyl; $C_2$-$C_6$-Alkenyl; Amino; $C_1$-$C_6$-Alkylamino; Di-$C_1$-$C_6$-alkylamino; Morpholino; Piperidino; Pyrazolidino; $C_1$-$C_8$-Alkylcarbonylamino;
Phenylamino oder Benzylamino bedeutet, wobei die aromatischen Ringe ihrerseits ein bis fünf Halogenatome und ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio oder wobei die aromatischen Ringe ihrerseits ein bis fünf Halogenatome oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;
Phenyl oder einen 5- bis 6-gliedrigen Heteroarylrest bedeutet, welcher ein bis drei Stickstoffatome und ein Sauerstoff- oder Schwefelatom enthalten kann oder ein Sauerstoff- oder Schwefelatom enthalten kann, wobei die aromatischen Reste ihrerseits ein bis fünf Halogenatome und ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio oder wobei die aromatischen Reste ihrerseits ein bis fünf Halogenatome oder ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^4$ für $C_1$-$C_8$-Alkyl; $C_1$-$C_8$-Halogenalkyl oder Phenyl steht, wobei der aromatische Rest seinerseits ein bis fünf Halogenatome und ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio, oder wobei der aromatische Rest seinerseits ein bis fünf Halogenatome oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^5$ für Wasserstoff; $C_1$-$C_4$-Alkyl oder Phenyl steht, wobei der aromatische Rest seinerseits ein bis fünf Halogenatome und ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio, oder wobei der aromatische Rest seinerseits ein bis fünf Halogenatome oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^6$ einen Phenylrest bedeutet, der ein bis fünf Halogenatome und ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro und Amino, oder der ein bis fünf Halogenatome oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro und Amino und

Z eine Ethylenbrücke bezeichnet, welche ihrerseits Teil eines 5- bis 6-gliedrigen Cycloalkyl- oder Cycloalkenylrestes sein kann, wobei dieser Rest ein bis vier Halogenatome und $C_1$-$C_4$-Alkylgruppen oder wobei dieser Rest ein bis vier Halogenatome oder $C_1$-$C_4$-Alkylgruppen tragen kann;
eine Ethenylenbrücke, welche Teil eines 5- bis 6-gliedrigen aromatischen oder heteroaromatischen Ringes sein kann, wobei diese Reste ihrerseits ein bis vier Halogenatome und ein bis zwei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio, oder wobei diese Reste ihrerseits ein bis vier Haolgenatome oder ein bis zwei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

wobei Z nicht für einen unsubstituierten Phenylrest steht, sowie deren landwirtschaftlich brauchbaren Salze.

Weiterhin betrifft die Erfindung Verfahren zur Herstellung der Verbindungen I sowie ihre Verwendung und die Verwendung von 2-N-Phthalimido-4-trichlormethylpyridin (Ia) als Nitrifikationsinhibitor.

Aus der DE-A 35 09 860 ist die Synthese von 2-Amino-4-trichlormethylpyridin (II) aus 2-N-Phthalimido-4-trichlormethylpyridin (Ia) und die Verwendung von II als Nitrifikationsinhibitor bekannt.

Nitrifikationsinhibitoren unterbinden für eine bestimmte Zeit im Boden die Oxidation von Ammonium zu Nitrat. Dies hat zur Folge, daß Nitrat während dieser Zeit weder von den Pflanzen aufgenommen und bei ungünstigen Bedingungen gespeichert noch in tiefere Bodenschichten verlagert und ins Grundwasser gelangen kann. Ammonium wird ebenfalls wie Nitrat von den Pflanzen aufgenommen und unterliegt als Kation im Boden der Sorption.

2-Amino-4-trichlormethylpyridin (II) läßt jedoch in seiner Wirkung zu wünschen übrig.

Der Erfindung lag die Aufgabe zugrunde Verbindungen zu finden und zu synthetisieren, die hinsichtlich der Wirkung als Nitrifikationshemmer verbesserte Eigenschaften haben.

Entsprechend dieser Aufgabe wurden die eingangs definierten 2-Amino-4-trichlorpyridinderivate I gefunden. Außerdem wurden Verfahren zur Herstellung dieser Verbindungen und Verfahren zur Hemmung der Nitrifikation mit diesen Verbindungen gefunden.

Die erfindungsgemäßen 2-Amino-4-trichlormethylpyridinderivate I sind auf verschiedenen Wegen zugänglich.

So erhält man beispielsweise die Verbindungen I, in denen $R^1$ Wasserstoff bedeutet, bevorzugt, indem man 2-Amino-4-trichlormethylpyridin II in an sich bekannter Weise (Houben-Weyl, Methoden der organischen Chemie, Bd. 8, S. 653-713 (1952)) in einem inerten aprotisch apolaren organischen Lösungsmittel in Gegenwart einer Base mit einem elektrophilen Reagens der Formel III umsetzt.

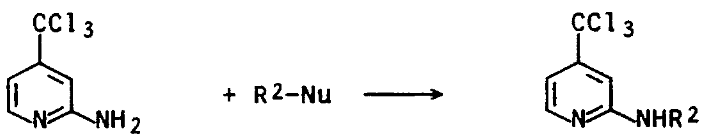

II III I ($R^1$=H)

Nu in der Formel III bedeutet eine nucleophile Abgangsgruppe wie Halogen, z.B. Fluor, Chlor, Brom und Jod, vorzugsweise Chlor und Brom.

Die Umsetzung wird im allgemeinen bei Temperaturen von 0° C bis 150° C, vorzugsweise 50° C bis 100° C durchgeführt.

Als Lösungsmittel eignen sich insbesondere Chlorbenzol, o-Dichlorbenzol und Nitrobenzol, wobei Basen wie Triethylamin oder Diisopropylethylamin eingesetzt werden, oder basische Lösungsmittel wie Pyridin oder Chinolin.

Üblicherweise setzt man die Edukte II und III in einem molaren Verhältnis von 1 bis 4 mol-Äquivalent, vorzugsweise 1 bis 2 mol-Äquivalent ein.

Man erhält die Verbindungen I auch dadurch, daß man 2-Chlor-4-trichlormethylpyridin IV in an sich bekannter Weise in einem inerten polaren Lösungsmittel mit einem Amin V umsetzt.

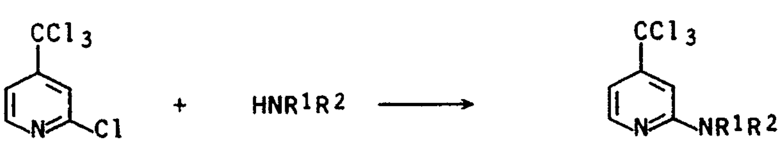

IV V I

Die Umsetzung wird im allgemeinen bei Temperaturen von 0° C bis 150° C, vorzugsweise 50° C bis

100 °C durchgeführt.

Als Lösungsmittel eignen sich insbesondere Chlorbenzol, o-Dichlorbenzol und Nitrobenzol, wobei Basen wie Triethylamin oder Diisopropylethylamin eingesetzt werden, oder basische Lösungsmittel wie Pyridin oder Chinolin.

Üblicherweise setzt man die Edukte IV und V in einem molaren Verhältnis von 1 bis 4 mol-Äquivalent, vorzugsweise 1 bis 2 mol-Äquivalent ein.

Verbindungen I, in denen $R^1$ und $R^2$ gemeinsam $=CR^5R^6$ bedeuten, erhält man bevorzugt, indem man 2-Amino-4-trichlorpyridin II in an sich bekannter Weise (Houben-Weyl, Methoden der organischen Chemie, Bd. 11/2, S. 73-98 (1958)) in einem inerten aprotisch apolaren organischen Lösungsmittel in Gegenwart eines Katalysators mit einem Aldehyd bzw. Keton VI kondensiert.

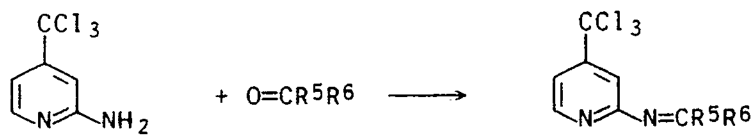

II VI I

Diese Umsetzung wird im allgemeinen bei Temperaturen von 20 °C bis 200 °C, vorzugsweise am Siedepnukt des Lösungsmittels durchgeführt.

Als Lösungsmittel eignen sich insbesondere Benzol, Toluol, Xylol, Chlorbenzol und Nitrobenzol.

Geeignete Katalysatoren sind Piperidiniumacetat, Ethyldiammoniumdiacetat, β-Alanin und p-Toluolsulfonsäure.

Üblicherweise werden die Edukte hierbei in einem Verhältnis von 1 mol-Äquivalent bis 4 mol-Äquivalent, vorzugsweise 1 mol-Äquivalent bis 1,5 mol-Äquivalent bezogen auf II eingesetzt.

Verbindungen I, in denen $R^1$ und $R^2$ gemeinsam -CO-Z-CO- bedeuten, erhält man bevorzugt, indem man 2-Amino-4-trichlormethylpyridin II in an sich bekannter Weise (Houben-Weyl, Methoden der organischen Chemie, Bd. 8, S. 656-657 (1952)) in einem inerten aprotisch apolaren organischen Lösungsmittel mit einem entsprechenden Anhydrid V unter Entzug des gebildeten Wassers kondensiert.

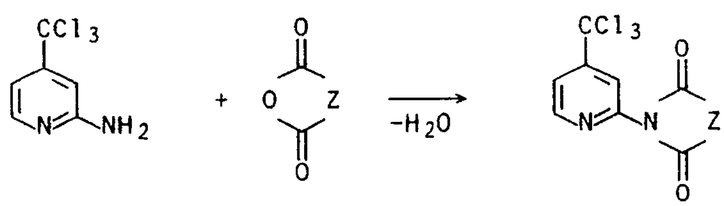


II V I

Die Umsetzung verläuft im allgemeinen bei Temperaturen von 50 °C bis 200 °C, vorzugsweise 100 °C bis 150 °C, bzw. bei dem Siedepunkt des Lösungsmittels.

Als Lösungsmittel eignen sich insbesondere Benzol, Toluol, Xylol, Chlorbenzol, o-Dichlorbenzol und Nitrobenzol.

Die Edukte werden üblicherweise in einem Verhältnis von 1 mol-Äquivalent bis 3 mol-Äquivalent, vorzugsweise 1 mol-Äquivalent bis 1,5 mol-Äquivalent, bezogen auf II umgesetzt.

Im Hinblick auf die bestimmungsgemäße Verwendung der 2-Amino-4-trichlormethylpyridine I als Nitrifikationsinhibitoren kommen als Substituenten vorzugsweise folgende Reste in Betracht:

$R^1$ Wasserstoff oder Alkyl wie Methyl, Ethyl, Propyl, 1-Methylethyl, Butyl, 1-Methylpropyl, 2-Methylpropyl, 1,1-Dimethylethyl, insbesondere Methyl;

$R^2$ Alkyl wie im allgemeinen und im besonderen bei $R^1$ genannt; Cycloalkyl wie Cyclopentyl und Cyclohexyl; Alkenyl wie Ethenyl, 1-Propenyl, 2-Propenyl, 1-Methylethenyl, 1-Butenyl, 2-Butenyl, 3-Butenyl, 1-Methyl-1-propenyl, 1-Methyl-2-propenyl, 2-Methyl-1-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 2-Propenyl; Phenyl, Benzyl oder Phenylsulfonyl, wobei die aromatischen Reste ihrerseits ein- bis fünffach durch Halogen wie Fluor, Chlor, Brom und Jod, insbesondere Chlor und Brom und/oder ein- bis dreifach durch folgende Gruppen substituiert sein können:

Alkyl mit 1 bis 4 Kohlenstoffatomen wie bei $R^1$ genannt, insbesondere Methyl;

Halogenalkyl, wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trichlormethyl;

Alkoxy wie Methoxy, Ethoxy, Propoxy, 1-Methylethoxy, Butoxy, 1-Methylpropoxy, 2-Methylpropoxy und 1,1-Dimethylethoxy, insbesondere Methoxy;

Halogenalkoxy, wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;

Methylthio, Ethylthio, Propylthio, 1-Methylethylthio, Butylthio, 1-Methylpropylthio, 2-Methylpropylthio und 1,1-Dimethylethylthio, insbesondere Butylthio;

ein Rest $-CX-R^3$,$-SO_2R^3$ oder $PX(OR^4)_2$ oder

$R^1$ und $R^2$ gemeinsam $=CR^5R^6$ oder -CO-Z-CO-;

X Sauerstoff oder Schwefel;

$R^3$ Alkyl wie bei $R^1$ genannt sowie Heptyl, 1-Methylhexyl, 2-Methylhexyl, 3-Methylhexyl, 4-Methylhexyl, 5-Methylhexyl, 1-Ethylpentyl, 2-Ethylpentyl, 3-Ethylpentyl, 4,4-Dimethylpentyl, 1-Ethyl-1-methylbutyl, Octyl, 1-Methylheptyl, 2-Methylheptyl, 3-Methylheptyl, 4-Methylheptyl, 5-Methylheptyl, 6-Methylheptyl, 1-Ethylhexyl, 2-Ethylhexyl, 3-Ethylhexyl, 4-Ethylhexyl, 5,5-Dimethylhexyl, Nonyl, 1-Methyloctyl, 2-Methyloctyl, 3-Methyloctyl, 4-Methyloctyl, 5-Methyloctyl, 6-Methyloctyl, 7-Methyloctyl, 1-Ethylheptyl, 2-Ethylheptyl, 3-Ethylheptyl, 4-Ethylheptyl, 5-Ethylheptyl, 6,6-Dimethylheptyl und Quindecyl, insbesondere 2,2-Dimethylpropyl;

Alkenyl, wie bei $R^2$ genannt, insbesondere 2-Propenyl;

Amino;

Alkylamino, wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 1,2-Dimethylpropylamino, 1,1-Dimethylpropylamino, 2,2-Dimethylpropylamino, 1-Ethylpropylamino, Hexylamino, 1-Methylpentylamino, 2-Methylpentylamino, 3-Methylpentylamino, 4-Methylpentylamino, 1,2-Dimethylbutylamino, 1,3-Dimethylbutylamino, 2,3-Dimethylbutylamino, 1,1-Dimethylbutylamino, 2,2-Dimethylbutylamino, 3,3-Dimethylbutylamino, 1,1,2-Trimethylpropylamino, 1,2,2-Trimethylpropylamino,1-Ethylbutylamino, 2-Ethylbutylamino und 1-Ethyl-2-methylpropylamino, insbesondere Butylamino;

Dialkylamino wie Dimethylamino, Diethylamino, Dipropylamino und Dibutylamino, insbesondere Diethylamino; Morpholino; Piperidino; Pyrazolidino;

Phenylamino oder Benzylamino, wobei die aromatischen Ringe ihrerseits ein bis fünf Halogenatome, wie insbesondere Fluor und Chlor und/oder ein bis drei der folgenden Gruppen tragen können: Alkyl, Halogenalkyl, Alkoxy, Halogenalkoxy und/oder Alkylthio mit jeweils 1 bis 4 Kohlenstoffatomen wie vorstehend genannt;

Alkylcarbonylamino wie Acetylamino, Propionylamino, Butyrylamino, 2-Methyl-propionylamino, Pentanoylamino, 2-Methylbutyrylamino, 3-Methylbutyrylamino, 2,2-Dimethylpropionylamino, Hexanoylamino, 2-Methylpentanoylamino, 3-Methylpentanoylamino, 4-Methylpentanoylamino, 2,2-Dimethylbutyrylamino, 2,3-Dimethylbutyrylamino, 3,3-Dimethylbutyrylamino und 2-Ethylbutyrylamino;
Phenyl oder Hetaryl wie Pyrrolyl, Pyrazolyl, Imidazolyl, Thiazolyl, Furyl, Isoxazolyl, Oxazolyl, Oxadiazolyl, Thienyl, Isothiazolyl, Thiazolyl, Thiadiazolyl, Pyridyl, Pyrimidyl, Pyrazinyl, Pyridazinyl und Triazinyl, wobei diese aromatischen Reste ihrerseits ein bis fünf Halogenatome, wie insbesondere Fluor und Chlor und/oder ein bis drei der folgenden Gruppen tragen kann: Alkyl, Halogenalkyl, Alkoxy, Halogenalkoxy und/oder Alkylthio mit jeweils 1 bis 4 Kohlenstoffatomen wie vorstehend genannt,

$R^4$ Alkyl mit 1 bis 8 Kohlenstoffatomen wie bei $R^3$ genannt, insbesondere Methyl und Ethyl;
Halogenalkyl, insbesondere wie bei $R^2$ genannt, oder Phenyl, wobei dieser aromatische Rest seinerseits ein bis fünf Halogenatome, wie insbesondere Fluor und Chlor und/oder ein bis drei der folgenden Gruppen tragen kann: Alkyl, Halogenalkyl, Alkoxy, Halogenalkoxy und/oder Alkylthio mit jeweils 1 bis 4 Kohlenstoffatomen wie vorstehend genannt;

$R^5$ Wasserstoff;
Alkyl mit 1 bis 4 Kohlenstoffatomen wie bei $R^1$ genannt, oder Phenyl, wobei dieser aromatische Rest seinerseits ein bis fünf Halogenatome, wie insbesondere Fluor und Chlor und/oder ein bis drei der folgenden Gruppen tragen kann: Alkyl, Halogenalkyl, Alkoxy, Halogenalkoxy und/oder Alkylthio mit jeweils 1 bis 4 Kohlenstoffatomen wie vorstehend genannt;

$R^6$ Phenyl, welches ein bis fünf Halogenatome wie insbesondere Fluor und Chlor und/oder ein bis drei der folgenden Reste tragen kann: Nitro, Amino, Alkyl, Halogenalkyl, Alkoxy, Halogenalkoxy oder Alkylthio mit jeweils 1 bis 4 Kohlenstoffatomen wie vorstehend genannt,
und

Z Ethylen, welches seinerseits Teil eines 5- bis 6-gliedrigen Cycloalkyl- oder Cycloalkenylrestes wie Cyclopentyl, Cyclohexyl, Cyclopentenyl und Cyclohexenyl sein kann, wobei dieser Rest ein bis vier Halogenatome, wie insbesondere Chlor und Brom und/oder ein bis drei Alkylgruppen mit jeweils 1 bis 4 Kohlenstoffatomen wie bei $R^1$ genannt, insbesondere Methyl tragen kann;
Ethenylen, welches Teil eines 5- bis 6-gliedrigen aromatischen oder heteroaromatischen Rings wie bei $R^3$ genannt sein kann, wobei dieser Rest seinerseits ein bis fünf Halogenatome, wie insbesondere Fluor und Chlor und/oder ein bis drei der folgenden Gruppen tragen kann: Alkyl, Halogenalkyl, Alkoxy, Halogenalkoxy und/oder Alkylthio mit jeweils 1 bis 4 Kohlenstoffatomen wie vorstehend genannt;
wobei Z nicht für einen unsubstituierten Phenylrest steht, sowie deren landwirtschaftlich brauchbaren Salze.

Die Wirkstoffe können auf die zu behandelnde Bodenfläche aufgebracht werden oder vor der Ausbringung mit Düngern vermischt werden.

Sofern die Wirkstoffe allein appliziert werden, empfehlen sich Aufwandmengen an Wirkstoff von 0,01 bis 20 kg/ha, vorzugsweise 0,1 bis 10 kg/ha und insbesondere 1 bis 5 kg/ha. Bei der gemeinsamen Ausbringung des Wirkstoffs mit Dünger wird die Aufwandmenge des Wirkstoffs durch die pro ha ausgebrachte Menge an Stickstoff im Dünger bestimmt. Im allgemeinen werden 0,01 bis 10 Gew.-% bezogen auf den Stickstoffaustrag pro ha zugemischt, vorzugsweise 0,1 bis 5 Gew.-% und insbesondere 1 bis 3 Gew.-%.

Die gemeinsame Ausbringung von Wirkstoff und formuliertem Dünger erfordert keine speziellen Formulierungen.

Synthesebeispiele:

Beispiel 1

2-Butyrylamino-4-trichlormethylpyridin

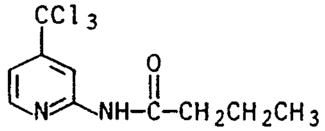

10,6 g (0,05 mol) 2-Amino-4-trichlormethylpyridin und 70 ml Pyridin wurden bei 0 °C unter Eiskühlung mit 7,3 ml (0,07 mol) Buttersäurechlorid versetzt. Nach dreistündigem Rühren bei 25 °C wurde das Reaktionsgemisch in 5 %ige Salzsäure gegeben und diese Mischung mit Ether extrahiert. Aus der organischen Phase erhielt man 12,7 g (90 %) des Produktes. Schmp.: 88 - 90 °C, Wirkstoffbeispiel 1.003

Beispiel 2

2-Methylaminocarbonylamino-4-trichlormethylpyridin

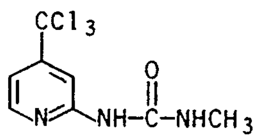

21,2 g (0,1 mol) 2-Amino-4-trichlormethylpyridin und 400 ml Toluol wurden bei 25 °C mit 17 ml (0.3 mol) Methylisocyanat und 1 ml Triethylamin versetzt. Nach 4 Stunden bei 80 °C wurde der entstandene Feststoff isoliert. Man erhielt 21,8 g (82 %) des gewünschten Produktes. Schmp.: 192 - 5 °C; Wirkstoffbeispiel 2.001

Beispiel 3

2-Diphenylphosponylamino-4-trichlormethylpyridin

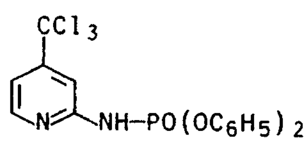

6,3 g (0,03 mol) 2-Amino-4-trichlormethylpyridin und 60 ml Pyridin wurden bei 0 °C mit 6,2 ml (0,03 mol) Phosphorsäurediphenylesterchlorid versetzt. Nach 12 Stunden bei 25 °C wurde die Reaktionsmischung in 200 ml 5 %ige Salzsäure gegeben. Der dabei gebildete farblose Niederschlag wurde isoliert. Man erhielt so 7,0 g (53 %)des gewünschten Produktes. Schmp.: 134 - 136 °C; Wirkstoffbeispiel 1.016

Beispiel 4

2-(2,3-Dichlorphenyl)methylenamino-4-trichlormethylpyridin

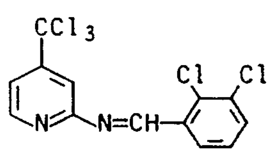

10,6 g (0,05 mol) 2-Amino-4-trichlormethylpyridin und 100 ml Toluol wurden mit 10,5 g (0,06 mol) 2,3-Dichlorbenzaldehyd und 1 g p-Toluolsulfonsäure 15 Stunden unter Abdestillieren von Wasser zum Sieden erhitzt. Anschließend wurde die so erhaltene Lösung mit Natriumsulfitlösung und mit Wasser gewaschen. Aus der organischen Phase erhielt man 13,7 g (75 %) des gewünschten Produkts. Schmp. 75 - 78 °C; Wirkstoffbeispiel 3.003

Beispiel 5

2-N-Succinimido-4-trichlormethylpyridin

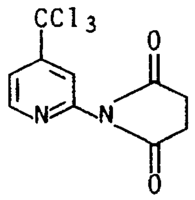

21,2 g (0,1 mol) 2-Amino-4-trichlormethylpyridin, 12,0 g (0,12 mol) Bernsteinsäureanhydrid und 500 ml 1,2-Dichlorbenzol wurden 2 Stunden unter Abdestillieren von Wasser zum Sieden erhitzt. Aus dieser Reaktionslösung erhielt man 19,0 g (65 %) des gewünschten Produktes. Schmp.: 139 °C; Wirkstoffbeispiel 4.001

Die in den vorstehenden Synthesebeispielen wiedergegebenen Arbeitsvorschriften wurden unter entsprechender Abwandlung der Edukte zur Herstellung weiterer Verbindungen I benutzt. Die so erhaltenen Verbindungen sind in den anschließenden Tabellen mit physikalischen Angaben aufgeführt.

Tabelle 1

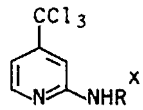

| Nr. | $R^x$ | phys. Daten [Fp (°C); NMR (δ in ppm)] |
|---|---|---|
| 1.001 | $COCH_3$ | 2,15 (s, 3H); 7,60 (m, 1H); 8,53 (d,1H); 8,72 (d, 1H); 11,0 (s, 1H)-$d_6$-DMSO |
| 1.002 | $COCH_2CH_3$ | 105 - 107 |
| 1.003 | $COCH_2CH_2CH_3$ | 88 - 90 |
| 1.004 | $COC(CH_3)_3$ | 76 - 78 |
| 1.005 | $COCH_2C(CH_3)_3$ | 116 - 118 |
| 1.006 | $COCH(CH_2CH_3)(CH_2)_3CH_3$ | 102 - 104 |
| 1.007 | $CO(CH_2)_{14}CH_3$ | 66 - 67 |
| 1.008 | $COC(CH_3)=CH_2$ | 111 - 112 |
| 1.009 | 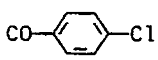 | 119 - 120 |
| 1.010 | 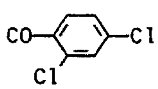 | 145 - 147 |
| 1.011 | 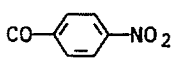 | 141 - 144 |
| 1.012 | 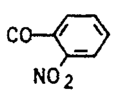 | 159 - 162 |
| 1.013 | 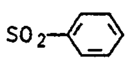 | 206 |
| 1.014 | 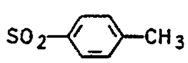 | 216 |
| 1.015 | 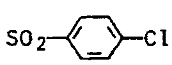 | 223 |
| 1.016 | $PO(OC_6H_5)_2$ | 134 - 136 |
| 1.017 | $PO(OCH_2CH_3)_2$ | 1,30 (m,6H); 4,00 (m,2H); 4,20 (m,2H); 6,98 (m,1H); 7,35 (m,1H); 7,85 (m,1H)–$CDCl_3$ |
| 1.018 | $PS(OCH_2CH_3)_2$ | 1,35 (m,6H); 4,25 (m, 4H); 7,60 (m,1H); 800 (m, 1H); 8,85 (m, 1H)–$CDCl_3$ |

Tabelle 2

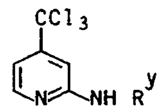

| Nr. | $R^y$ | phys. Daten [Fp (°C); NMR (δ in ppm)] |
|---|---|---|
| 2.001 | $CONHCH_3$ | 192 - 195 |
| 2.002 | $CONH(CH_2)_3CH_3$ | 81 - 82 |
| 2.003 | 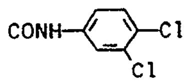 | > 240 |
| 2.004 | $CSNH_2$ | 207 |
| 2.005 | 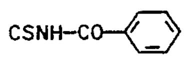 | 148 |

Tabelle 3

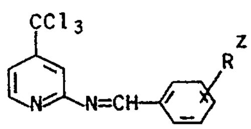

| Nr. | $R^z$ | phys. Daten [Fp (°C); NMR (δ in ppm)] |
|---|---|---|
| 3.001 | H | 130 - 132 |
| 3.002 | 4-Cl | 129 - 131 |
| 3.003 | 2,3-Cl,Cl | 75 - 78 |
| 3.004 | 2,4-Cl,Cl | 105 - 107 |
| 3.005 | 2,6-Cl,Cl | 7,35 (m,1H); 7,48 (m,1H); 7,70 (m,1H); 7,85 (m,1H); 8,30 (d,1H); 8,65 (d,1H); 9,62 (s,1H)-$CDCl_3$ |
| 3.006 | 3,4-Cl,Cl | 73 - 75 |
| 3.007 | $4-OCH_3$ | 3,90 (s,3H); 7,85 (d,2H); 7,95 (d,2H); 8,15 (d,1H); 8,58 (d,1H); 9,12 (s,1H); 9,88 (s, 1H)-$CDCl_3$ |
| 3.008 | $4-NO_2$ | 138 - 139 |

Tabelle 4

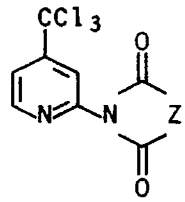

| Nr. | Z | phys. Daten [Fp (°C); NMR (δ in ppm)] |
|---|---|---|
| 4.001 | $-CH_2CH_2-$ | 139 |
| 4.002 | 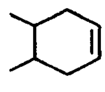 | 140 |
| 4.003 | 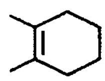 | 90 - 92 |
| 4.004 | 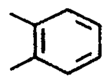 | 138 - 140 |
| 4.005 | 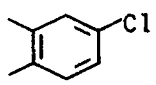 | 66 - 69 |
| 4.006 | 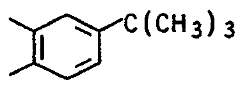 | 105 - 110 |

Anwendungsbeispiele

Untersuchung der Hemmung der Nitrifikation im Boden und der Reduktion des Nitratgehaltes in Pflanzen

Im nachstehenden wird ein Vergleich der Nitrifikationswirkung der erfindungsgemäßen Verbindung Nr. 1.004 und des bekannten Nitrifikationsinhibitors A (2-Amino-4-trichlormethylpyridin) wiedergegeben.

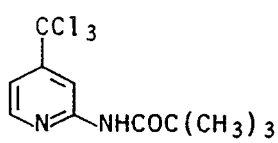

Beispiel Nr. 1.004

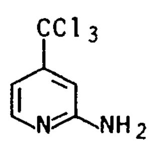

A

Bei den Versuchen wurde jeweils 1 ppm Wirkstoff und 220 mg Ammoniumsulfat (= 46,6 mg N) zu 200 g eines auf 50 % der maximalen Wasserkapazität (WK max.) eingestellten Bodens gemischt und zur Bebrütung bei 20 °C aufgestellt. Nach vier, sechs und acht Wochen wurde das im Boden noch vorhandene Ammonium bestimmt, und die Ergebnisse relativ zu der zu Beginn eingesetzten Menge ausgedrückt.

Die Versuche zur Reduktion des Nitratgehaltes in pflanzlichem Material wurden in Mitscherlichgefäßen, die 6,5 kg Boden fassen, durchgeführt. Der Boden wurde mit 1 g N als Ammonsulfat plus 1 % Wirkstoff (bezogen auf die eingesetzte N-Menge) vermischt und im Gefäß während der gesamten Versuchsdauer bei zunächst 40 % der WK max. und nach Keimen und Anwachsen der Pflanzen bei 60 % der WK max. gehalten. Nach Erreichen der verzehrfähigen Reife (technische Reife) wurden die Pflanzen geerntet und der Nitratgehalt bestimmt. Die Ergebnisse wurden in ppm $NO_3$ auf Frischmasse bezogen angegeben. Als Beispielpflanze wurde Spinat verwendet.

Die Ergebnisse über die Beeinflussung der Nitrifikation im Boden sind der Tabelle A zu entnehmen. Daraus geht hervor, daß das Beispiel Nr. 1.004 eine Hemmung der Nitrifikation von 90 % über einen Zeitraum von acht Wochen bewirkte. Die Vergleichsverbindung A erreichte über den gleichen Zeitraum

lediglich eine Hemmleistung von 58 %. Ohne Wirkstoff war nach vier Wochen bereits 90 % des $NH_4$-N umgesetzt.

Die Reduktion des Nitratgehaltes in Spinat geht aus Tabelle B hervor. Ausgehend von einem Nitratgehalt von 1203 ppm (Frühjahr) bzw. 4203 ppm (Herbst) bewirkten beide Substanzen eine deutliche Verminderung der Nitratwerte. Dennoch war die Reduktion durch das Beispiel Nr. 1.004 ausgeprägter. Die sehr niedrigen Werte des Versuches ohne N sind belanglos, da hierbei sehr niedrige Pflanzenerträge von nicht marktfähiger Ware produziert wurden.

Tabelle A: Hemmung der Nitrifikation von Ammonium-N durch Zugabe von 1 ppm Wirkstoff zum Boden

| Beispiel Nr. | Wirkungsdauer (%) 4 | 6 | 8 Wochen |
|---|---|---|---|
| A | 95 | 94 | 58 |
| 1.004 | 100 | 95 | 90 |
| ohne Wirkstoff | 10 | 0 | 0 |

Tabelle B: Der Einfluß von Nitrifikationshemmern auf den Nitratgehalt von Spinat

| Beispiel Nr. | N-Zugabe | Aufwandmenge an Wirkstoff/N | ppm $NO_3$ [bezogen auf die Frischmasse] Frühjahr | Herbst |
|---|---|---|---|---|
| - | - | - | 36 | 121 |
| - | + | - | 1203 | 4203 |
| A | + | 1 % | 424 | 1927 |
| 1.004 | + | 1 % | 71 | 934 |

-: kein Ammoniumsulfat
+: 1 g N als Ammoniumsulfat

## Patentansprüche

1. 2-Amino-4-trichlormethylpyridine der allgemeinen Formel I

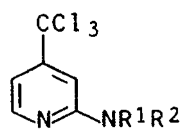

I

in der die Substituenten folgende Bedeutung haben:

$R^1$ Wasserstoff oder eine $C_1$-$C_8$-Alkylgruppe;

$R^2$ eine $C_1$-$C_8$-Alkylgruppe; eine $C_5$-$C_8$-Cycloalkylgruppe; eine $C_2$-$C_4$-Alkenylgruppe; einen Phenyl-, Benzyl- oder Phenylsulfonylrest, wobei die aromatischen Reste ihrerseits ein- bis fünffach durch Halogen und ein- bis dreifach durch folgende Gruppen substituiert sein können: $C_1$-$C_4$-Alkyl, $C_1$-$C_4$-Halogenalkyl, $C_1$-$C_4$-Alkoxy, $C_2$-$C_4$-Halogenalkoxy und $C_1$-$C_4$-Alkylthio, oder wobei die aromatischen Reste ihrerseits ein- bis

fünffach durch Halogen oder ein- bis dreifach durch folgende Gruppen substituiert sein können: $C_1$-$C_4$-Alkyl, $C_1$-$C_4$-Halogenalkyl, $C_1$-$C_4$-Alkoxy, $C_2$-$C_4$-Halogenalkoxy und $C_1$-$C_4$-Alkylthio;

einen Rest -CX-$R^3$, -$SO_2R^3$ oder -$PX(OR^4)_2$ oder

$R^1$ und $R^2$ gemeinsam $=CR^5R^6$ oder -CO-Z-CO-, wobei

X für Sauerstoff oder Schwefel steht,

$R^3$ $C_1$-$C_{20}$-Alkyl; $C_2$-$C_6$-Alkenyl; Amino; $C_1$-$C_6$-Alkylamino; Di-$C_1$-$C_6$-alkylamino; Morpholino; Piperidino; Pyrrazolidino; $C_1$-$C_8$-Alkylcarbonylamino;
Phenylamino oder Benzylamino bedeutet, wobei die aromatischen Ringe ihrerseits ein bis fünf Halogenatome und ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio oder wobei die aromatischen Ringe ihrerseits ein bis fünf Halogenatome oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;
Phenyl oder einen 5- bis 6-gliedrigen Heteroarylrest bedeutet, welcher ein bis drei Stickstoffatome und ein Sauerstoff- oder Schwefelatom enthalten kann oder ein Sauerstoff- oder Schwefelatom enthalten kann, wobei die aromatischen Reste ihrerseits ein bis fünf Halogenatome und ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio oder wobei die aromatischen Reste ihrerseits ein bis fünf Halogenatome oder ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^4$ für $C_1$-$C_8$-Alkyl; $C_1$-$C_8$-Halogenalkyl oder Phenyl steht, wobei der aromatische Rest seinerseits ein bis fünf Halogenatome und ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio, oder wobei der aromatische Rest seinerseits ein bis fünf Halogenatome oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^5$ für Wasserstoff; $C_1$-$C_4$-Alkyl oder Phenyl steht, wobei der aromatische Rest seinerseits ein bis fünf Halogenatome und ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio, oder wobei der aromatische Rest seinerseits ein bis fünf Halogenatome oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^6$ einen Phenylrest bedeutet, der ein bis fünf Halogenatome und ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro und Amino, oder der ein bis fünf Halogenatome oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro und Amino und

Z eine Ethylenbrücke bezeichnet, welche ihrerseits Teil eines 5- bis 6-gliedrigen Cycloalkyl- oder Cycloalkenylrestes sein kann, wobei dieser Rest ein bis vier Halogenatome und $C_1$-$C_4$-Alkylgruppen oder wobei dieser Rest ein bis vier Halogenatome oder $C_1$-$C_4$-Alkylgruppen tragen kann;
eine Ethenylenbrücke, welche Teil eines 5- bis 6-gliedrigen aromatischen oder heteroaromatischen Ringes sein kann, wobei diese Reste ihrerseits ein bis vier Halogenatome und ein bis zwei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio, oder wobei diese Reste ihrerseits ein bis vier Haolgenatome oder ein bis zwei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

wobei Z nicht Teil eines unsubstituierten Phenylrestes ist, sowie deren landwirtschaftlich brauchbaren Salze.

**2.** Verfahren zur Herstellung der Verbindungen I gemäß Anspruch 1, in denen $R^1$ Wasserstoff und $R^2$ nicht Phenyl bedeutet, dadurch gekennzeichnet, daß man 2-Amino-4-trichlormethylpyridin II

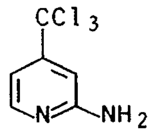

II

in an sich bekannter Weise in einem inerten organischen Lösungsmittel in Gegenwart einer Base mit einem elektrophilen Reagens III

$R^2$-Nu     III

in der Nu eine nucleophile Abgangsgruppe bedeutet, umsetzt.

3. Verfahren zur Herstellung der Verbindungen I gemäß Anspruch 1, dadurch gekennzeichnet, daß man 2-Chlor-4-trichlormethylpyridin IV

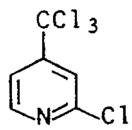

IV

in an sich bekannter Weise in einem inerten organischen Lösungsmittel mit einem Amin V

$HNR^1R^2$     V

umsetzt.

4. Verfahren zur Herstellung der Verbindungen I gemäß Anspruch 1, in denen $R^1$ und $R^2$ gemeinsam $=CR^5R^6$ bedeuten dadurch gekennzeichnet, daß man 2-Amino-4-trichlormethylpyridin II gemäß Anspruch 2 in an sich bekannter Weise in einem inerten organischen Lösungsmittel in Gegenwart eines Katalysators mit einem Aldehyd bzw. Keton VI

$O=CR^5R^6$     VI

kondensiert.

5. Verfahren zur Herstellung der Verbindungen I gemäß Anspruch 1, in denen $R^1$ und $R^2$ gemeinsam -CO-Z-CO- bedeuten, dadurch gekennzeichnet, daß man 2-Amino-4-trichlormethylpyridin II gemäß Anspruch 2 in an sich bekannter Weise in einem inerten organischen Lösungsmittel mit einem entsprechenden Anhydrid VII

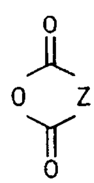

VII

kondensiert.

6. Verfahren zur Hemmung der Nitrifikation des Bodens, dadurch gekennzeichnet, daß man eine wirksame Menge eines 2-Amino-4-trichlormethylpyridins der allgemeinen Formel I gemäß Anspruch 1 oder eine wirksame Menge von 2-N-Phthalimido-4-trichlormethylpyridin (Ia) auf den Boden appliziert.